(19) Europäisches Patentamt
European Patent Office
Office européen des brevets

(11) Veröffentlichungsnummer : **0 485 855 B1**

(12) **EUROPÄISCHE PATENTSCHRIFT**

(45) Veröffentlichungstag der Patentschrift : **08.06.94 Patentblatt 94/23**

(51) Int. Cl.$^5$ : **C07C 68/06,** C07C 69/96

(21) Anmeldenummer : **91118803.5**

(22) Anmeldetag : **05.11.91**

(54) **Verfahren zur Herstellung von aromatischen Kohlensäurediestern.**

(30) Priorität : **16.11.90 DE 4036594**

(43) Veröffentlichungstag der Anmeldung : **20.05.92 Patentblatt 92/21**

(45) Bekanntmachung des Hinweises auf die Patenterteilung : **08.06.94 Patentblatt 94/23**

(84) Benannte Vertragsstaaten : **BE DE ES FR GB IT NL**

(56) Entgegenhaltungen : **JP-A-54 125 617**

(73) Patentinhaber : **BAYER AG**
**D-51368 Leverkusen (DE)**

(72) Erfinder : **Schön, Norbert, Dr.**
**Wilhelmshofsallee 82**
**W-4150 Krefeld (DE)**
Erfinder : **Buysch, Hans-Josef, Dr.**
**Brandenburger Strasse 28**
**W-4150 Krefeld (DE)**
Erfinder : **Zirngiebl, Eberhard, Dr.**
**Roggendorfstrasse 65**
**W-5000 Köln 80 (DE)**
Erfinder : **Kischkewitz, Jürgen, Dr.**
**Vogelsangweg 38**
**W-4030 Ratingen 6 (DE)**

EP 0 485 855 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von aromatischen Kohlensäurediestern durch katalysierte Umesterung, wobei als Katalysatoren Titandioxide verwendet werden.

Die Herstellung von aromatischen und aliphatisch-aromatischen Kohlensäurediestern durch Umesterung ausgehend von aliphatischen Kohlensäureestern und Phenolen ist prinzipiell bekannt. Dabei handelt es sich um eine Gleichgewichtsreaktion, wobei die Lage des Gleichgewichts fast vollständig in Richtung der aliphatisch substituierten Kohlensäureester verschoben ist. Daher ist es verhältnismäßig leicht, aus aromatischen Kohlensäureestern und Alkoholen aliphatische Kohlensäurediester herzustellen, während die Reaktion im umgekehrten Sinn nur dann gut gelingt, wenn sehr reaktive und selektive Katalysatoren zur Verfügung stehen.

Für den genannten Zweck sind eine Vielzahl von homogen löslichen oder während der Reaktion homogen in Lösung gehenden Katalysatoren bekannt, beispielsweise Lewissäure-Katalysatoren aus der Gruppe der Metallhalogenide oder entsprechender Acyloxy-, Alkoxy-und Aryloxy-Verbindungen von Al, Ti, U, V, Zn, Fe und Sn (DE-OS 25 28 412 und 25 52 907) oder Zinnverbindungen der Formel $-R_2SnO-$ (DE-OS 34 45 552).

Es bestand der Wunsch, heterogene Katalysatoren zu finden, die genügend reaktiv und selektiv beim erfindungsgemäßen Umesterungsprozeß wirken. Solche heterogen oder festphasengebundene Umesterungskatalysatoren hätten den Vorteil, daß sie nach Beendigung der Reaktion problemlos von den Produkten abgetrennt werden könnten bzw. vorteilhaft in einem kontinuierlich arbeitenden Prozess eingesetzt werden könnten.

Heterogene Umesterungskatalysatoren für die genannte Umesterung aus Mischoxiden von Silicium und Titan, durch gemeinsame Hydrolyse von Silicium- und Titanhalogeniden hergestellt, sind bekannt (JP 54/125,617 (1979)). Diese Katalysatoren haben jedoch den Nachteil, daß sie zu unselektiv wirken und einen beträchtlichen Anteil an Decarboxylierungsprodukten z.B. Alkylaryl und Diarylether bilden. Reine Titandioxide, die hauptsächlich für Pigmentanwendungen eingesetzt werden, sind zwar wesentlich selektiver als Mischoxide von Silicium und Titan, jedoch nur sehr wenig aktiv.

Es wurde nun gefunden, daß mit Titanoxiden, die hohe innere Oberflächen besitzen, eine schnelle und dennoch selektive Umesterung im gewünschten Sinne erreicht werden kann. Es wird praktisch kein Kohlendioxid abgespalten, die Aktivität der Katalysatoren bleibt auch nach mehrfachem Gebrauch erhalten.

Die Erfindung betrifft also ein Verfahren zur Herstellung von mindestens eine aromatische Estergruppe enthaltenden Kohlensäurediestern aus mindestens eine aliphatische Estergruppe enthaltenden Kohlensäurediestern durch katalysierte Umesterung mit einen Phenol, das dadurch gekennzeichnet ist, daß als Katalysator ein Titandioxid mit einer nach der BET-Methode bestimmten Oberfläche von mindestens 20 $m^2/g$ in einer Menge von 0,1-200 Gew.-%, bezogen auf den eingesetzten, mindestens eine aliphatische Estergruppe enthaltenden Kohlensäurediester, eingesetzt wird.

Es können dabei Titandioxide in verschiedenen Kristallmodifikationen eingesetzt werden, z.B. Anatase und Rutile, wobei bevorzugt Anatase benutzt werden. Es ist außerdem möglich, mit verschiedenen Metalloxiden, z.B. solchen von Al, Zr, Ce, Nb, V, Sb, W und Mo, sowie mit Alkalimetallen dotierte Titandioxide einzusetzen. Die verwendeten Titandioxidkatalysatoren besitzen eine nach der BET- Methode bestimmte Oberfläche von mindestens 20 $m^2/g$, bevorzugt von mindestens 50 $m^2/g$ und besonders bevorzugt von mindestens 90 $m^2/g$. Die Obergrenze der Oberfläche kann bis zu 1000 $m^2/g$ reichen, in der Praxis vielfach bis 500 $m^2/g$. Für manche Anwendungszwecke ist es zweckmäßig und wünschenswert, Titandioxide, die an feste Träger gebunden sind, einzusetzen. Dabei können nur Trägersubstanzen, z.B. Edelstahlplatten, Wabenkörper, Streckmetalle, Siebe, verwendet werden, die die Selektivität und die innere Oberfläche des Titandioxides nicht negativ beeinflussen. Die erfindungsgemäß einzusetzenden Titandioxide werden nach üblichen Verfahren aus Titanhaltigen Rohstoffen, beispielsweise mit Hilfe des Sulfatprozesses, hergestellt.

Die erfindungsgemäßen Titandioxid-Katalysatoren werden in Mengen von 0,1 - 200 Gew.-%, bevorzugt 1 - 100 Gew.-% und besonders bevorzugt 2 - 50 Gew.-%, bezogen auf eingesetzten, mindestens eine aliphatische Estergruppe enthaltenden Kohlensäurediester, verwendet.

Als erfindungsgemäße katalysierte Umesterung ist der Austausch einer oder zweier aliphatischer Estergruppen durch eine oder zwei aromatische Estergruppen zu verstehen. Dies kann beispielsweise die Umsetzung eines aliphatisch-aromatischen Kohlensäurediesters mit einem Phenol zu einem rein aromatischen Kohlensäurediester, gegebenenfalls mit zwei unterschiedlichen aromatischen Estergruppen, sein. Es kann weiterhin die Umsetzung eines rein aliphatischen Kohlensäurediesters mit einem Phenol zu einem aliphatisch-aromatischen oder zu einem rein aromatischen Kohlensäurediester sein. In diesem Fall könnte zunächst, etwa durch Begrenzung der Phenolmenge, eine aliphatische Estergruppe ausgetauscht und danach, gegebenenfalls durch ein unterschiedliches Phenol, die zweite aliphatische Estergruppe ausgetauscht werden, so daß auch hier Kohlensäureester mit zwei verschiedenen aromatischen Estergruppen erhältlich sind.

In den genannten Fällen wird der leichter flüchtige aliphatische Esteralkohol destillativ aus dem Umsetzungsgemisch entfernt. Schließlich kann die erfindungsgemäße katalysierte Umesterung auch die Disproportionierung eines gemischten aliphatisch-aromatischen Kohlensäurediesters zu einem rein aromatischen Kohlensäurediester und einem destillativ ebenfalls abtrennbaren rein aliphatischen Kohlensäurediester sein.

Als Phenol zur Umesterung kann auch ein Bisphenol eingesetzt werden.

Für den Fall der Umesterung unter Einsatz eines Phenols kann das Gewichtsverhältnis zwischen dem eingesetzten mindestens eine aliphatische Estergruppe enthaltenden Kohlensäurediester und einem solchen Phenol in weiten Grenzen variiert werden, beispielsweise von 1:99 bis 99:1, bevorzugt 1:9 bis 9:1. Dabei wird im Falle eines großen Überschusses an Phenol hauptsächlich der Diarylkohlensäurediester, im Fall eines großen Überschusses an rein aliphatischem Kohlensäurediester, vorzugsweise der gemischte aliphatisch-aromatische Kohlensäurediester gebildet. Bei Verwendung von Bisphenolen und mindestens 2 Äquivalenten Kohlensäurediester werden Biscarbonate gebildet, die an den Molekülenden noch aliphatische oder aromatische Monoestergruppen aufweisen. Beim Einsatz etwa äquivalenter Mengen Bisphenol und Kohlensäurediester erhält man zunächst Monocarbonate mit einer freien phenolischen Hydroxylgruppe des Bisphenols. Unter verschärften Reaktionsbedingungen entstehen hieraus oligomere oder polymere aromatische Carbonate. Die verschiedenartig substituierten Kohlensäurediester können problemlos, beispielsweise durch Destillation, voneinander getrennt werden.

Erfindungsgemäß einzusetzende mindestens eine aliphatische Estergruppe enthaltende Kohlensäurediester sind solche der Formel

$$R^1O\text{-}CO\text{-}OR^2 \qquad (I),$$

worin

$R^1$ und $R^2$ unabhängig voneinander geradkettiges oder verzweigtes $C_1$-$C_{12}$-Alkyl oder $C_3$-$C_8$-Cycloalkyl bedeuten, wobei weiterhin $R^1$ substituiertes oder nicht substituiertes $C_6$-$C_{12}$-Aryl bedeuten kann.

In bevorzugter Form werden Kohlensäurediester der Formel

$$R^3O\text{-}CO\text{-}OR^4 \qquad (II)$$

eingesetzt, in der

$R^3$ und $R^4$ unabhängig voneinander geradkettiges oder verzweigtes $C_1$-$C_8$-Alkyl, Cyclopropyl, Cyclopentyl oder Cyclohexyl bedeuten, wobei weiterhin $R^3$ substituiertes oder nicht substituiertes Phenyl bedeuten kann.

Geradkettiges oder verzweigtes $C_1$-$C_{12}$-Alkyl ist beispielsweise: Methyl, Ethyl, Propyl, Isopropyl, Butyl, Isobutyl, t-Butyl, die isomeren Pentyle, Hexyle, Octyle (u.a. 2-Ethyl-hexyl), Decyle oder Dodecyle, bevorzugt eines der $C_1$-$C_8$-Alkyle, besonders bevorzugt eines der $C_1$-$C_4$-Alkyle.

$C_3$-$C_8$-Cycloalkyl ist beispielsweise Cyclopropyl, Cyclopentyl, Methyl-cyclopentyl, Cyclohexyl, Methylcyclohexyl, Cycloheptyl oder Cyclooctyl, bevorzugt Cyclopropyl, Cyclopentyl oder Cyclohexyl.

$C_6$-$C_{12}$-Aryl ist beispielsweise Phenyl, Biphenylyl oder Naphthyl, bevorzugt Phenyl. Für den Fall einer Substitution von $C_6$-$C_{12}$-Aryl kommen ein oder zwei Substituenten aus der Gruppe $C_1$-$C_4$-Alkyl, $C_1$-$C_4$-Alkoxy, Cyano, Fluor, Chlor oder Brom (Chlor ist bevorzugtes Halogen) in Betracht.

Wichtige einzusetzende zwei aliphatische Estergruppen enthaltende Kohlensäurediester sind beispielsweise: Dimethylcarbonat, Diethylcarbonat, Dibutylcarbonat, Diisopropylcarbonat, Dicyclohexylcarbonat, Dioctylcarbonat, bevorzugt Dimethyl- und Diethylcarbonat.

Wichtige einzusetzende aliphatisch-aromatische Kohlensäurediester sind beispielsweise:
Methyl-phenyl-carbonat, Ethyl-phenyl-carbonat, Butyl-phenyl-carbonat, Methyl-kresyl-carbonat und ihre Homologen.

Für den Fall, daß die Umesterung mit Hilfe eines Phenols vorgenommen wird, wird ein solches der Formel

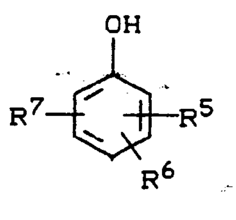
(III)

eingesetzt, in der

$R^5$ Wasserstoff, $C_1$-$C_4$-Alkyl, $C_2$-$C_4$-Alkenyl, $C_1$-$C_4$-Alkoxy, $C_5$-$C_6$-Cycloalkyl, Phenyl, Fluor, Chlor, Brom oder Cyano bedeutet;

$R^6$ für Wasserstoff, $C_1$-$C_4$-Alkyl, $C_2$-$C_4$-Alkenyl, $C_1$-$C_4$-Alkoxy, Fluor, Chlor oder Brom steht und

$R^7$ Wasserstoff, $C_1$-$C_4$-Alkyl oder die Gruppe

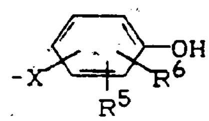

darstellt, in der X eine Einfachbindung, $-CH_2-$, $C_2$-$C_5$-Alkylen, $C_2$-$C_5$-Alkyliden, $C_5$-$C_6$-Cycloalkylen, $C_5$-$C_{10}$-Cycloalkyliden, Sauerstoff, Schwefel, -CO-, -SO- oder $-SO_2-$ bedeutet,
wobei $R^6$ und $R^7$ gemeinsam auch einen anellierten Benzolkern bedeuten können.

Alkylengruppen sind über zwei verschiedene C-Atome mit den aromatischen Kernen verbunden, also in 1,2-, 1,3-, 1,4-, 1,5-, 2,3- oder 2,4-Verknüpfung; Alkylidengruppen sind über das gleiche C-Atom mit den aromatischen Kernen verbunden, also in 1,1-, 2,2- oder 3,3-Verknüpfung. Cycloalkylen und Cycloalkyliden können 1 bis 3-fach durch Methyl oder Ethyl substituiert sein.

Als Monophenole werden zur Umesterung in bevorzugter Weise solche der Formel

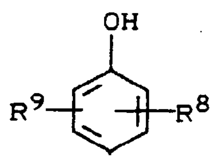

(IV)

eingesetzt, in der

| | |
|---|---|
| $R^8$ und $R^9$ | unabhängig voneinander Wasserstoff, $C_1$-$C_4$-Alkyl oder $C_5$-$C_6$-Cycloalkyl, Phenyl oder Chlor bedeuten kann. |

Als Phenole seien beispielhaft genannt:
Nicht substituiertes Phenol, o-, m- oder p-Kresol, o-, m- oder p-Chlorphenol, o-, m- oder p-Ethylphenol, o-, m- oder p-Propylphenol, 2,6- Dimethylphenol, 2,4-Dimethylphenol und 3,4-Dimethylphenol.

Bevorzugte Bisphenole sind solche der Formel

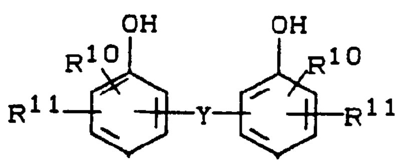

(V)

in der

| | |
|---|---|
| $R^{10}$ und $R^{11}$ | unabhängig voneinander Wasserstoff, $C_1$-$C_4$-Alkyl, $C_2$-$C_4$-Alkenyl, $C_1$-$C_4$-Alkoxy, $C_5$-$C_6$-Cycloalkyl, Fluor, Chlor oder Brom bedeuten kann, und |
| Y | für eine Einfachbindung, $-CH_2-$, $-C_2$-$C_5$-Alkyliden, $C_5$-$C_{10}$-Cycloalkyliden, Schwefel oder $-SO_2-$ steht. |

Als Bisphenole werden zur Umesterung in besonders bevorzugter Weise solche eingesetzt, in denen X bzw. Y und die Hydroxylgruppen in o-,o'-, p-,p'- oder o-,p'-Stellung zueinander stehen.

Ganz besonders bevorzugte Bisphenole sind solche der Formeln

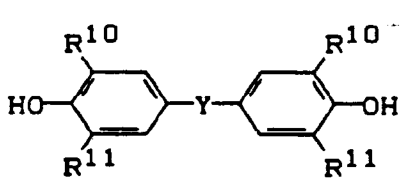

bzw.

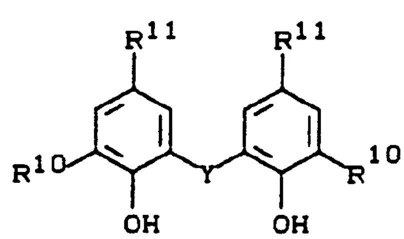

(VI) (VII)

bzw.

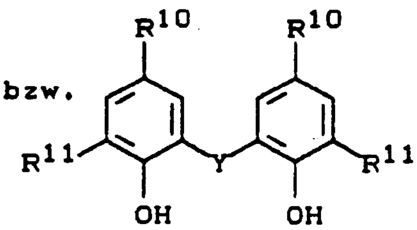

(VIII)

in denen $R^{10}$, $R^{11}$ und Y die obengenannte Bedeutung haben.

Als Bisphenole seien beipielsweise genannt:

2,2-Bis(4-hydroxyphenyl)-propan (=Bisphenol A), Bis(4-hydroxyphenyl)-methan, 1,1-Bis(4-hydroxyphenyl)-cyclohexan, 1,1-Bis(4-hydroxyphenyl)-3,5,5-trimethylcyclohexan, 2,2-Bis(3,5-dimethyl-4-hydroxyphenyl)-propan, 2,2'- und 4,4'-Dihydroxy-biphenyl, Bis(5-methyl-2-hydroxyphenyl)-methan, Bis(3,5-dimethyl-2-hydroxyphenyl)-methan, Bis(3-tert.-butyl-5-methyl-2-hydroxyphenyl)-methan. Besonders wichtig unter diesen ist das Bisphenol A.

Erfindungsgemäß erhältliche mindestens eine aromatische Estergruppe enthaltende Kohlensäurediester sind solche der Formel

$$R^{12}O\text{-}CO\text{-}OR^{13} \qquad (IX)$$

in der

$R^{12}$ und $R^{13}$ unabhängig voneinander substituiertes oder nicht substituiertes $C_6$-$C_{12}$-Aryl bedeutet, das in der oben angegebenen Weise substituiert sein kann, wobei weiterhin $R^{15}$ geradkettiges oder verzweigtes $C_1$-$C_{12}$-Alkyl oder $C_3$-$C_8$-Cycloalkyl bedeuten kann.

Das erfindungsgemäße Verfahren wird bei einer Temperatur von 50-300°C, bevorzugt 100-250°C, durchgeführt. Der Druck ist grundsätzlich nicht kritisch und kann im weiten Bereich von 0,1-50 bar, bevorzugt 1-20 bar, besonders bevorzugt 1-15 bar, liegen.

Erfindungsgemäß kann ohne jedes Lösungsmittel, also in der Schmelze der umzusetzenden Stoffe, gearbeitet werden. Es ist jedoch gleichermaßen möglich, in einem bezüglich der Reaktion inerten Lösungsmittel zu arbeiten. Die Arbeitsweise unter Benutzung eines solchen inerten Lösungsmittels kann beispielsweise dann von Bedeutung sein, wenn der durch die Umesterung entstehende Alkohol mit Hilfe eines solchen Lösungsmittels leichter aus dem Reaktionsgemisch entfernt werden kann. Lösungsmittel die erfindungsgemäß geeignet sind, sind beispielsweise:

Aromatische (Halogen)Kohlenwasserstoffe, wie Toluol, Xylole, Chlorbenzol, Dichlorbenzole, Trimethylbenzole, Biphenyl, und (cyclo)aliphatische Kohlenwasserstoffe, wie Hexan, Heptan, Isooctan, Cyclohexan, Dekalin, Ligroin, Petrolether, sowie aliphatische und aromatische Nitrile und Ketone, wie Aceton, Acetonitril, Acetylbenzol, Benzonitril und andere.

Eine mögliche Verfahrensweise besteht darin, daß das Umesterungsgemisch in einer Apparatur mit einer ausreichenden Kolonne auf die gewünschte Reaktionstemperatur gebracht wird und der herausspaltende aliphatische Alkohol über Kopf abdestilliert wird, Für den Fall einer Disproportionierung, bei der aus einem aliphatisch-aromatischen Kohlensäurediester der vollständig aromatische Kohlensäurediester und der vollständig aliphatische Kohlensäurediester entstehen, kann der im allgemeinen leichter flüchtige vollständig aliphatische Kohlensäurediester über Kopf abdestilliert werden. Die Abtrennung des leichter flüchtigen Stoffes kann in der beschriebenen Weise unter Benutzung eines inerten Lösungsmittels oder Gasstromes erfolgen. Weiterhin ist es möglich, einen oder beide Ausgangsstoffe nur zum Teil vorzulegen und nach Anlaufen der Reaktion in die Schmelze oder Lösung des Reaktionsgemisches nachzudosieren.

Für den Fall, daß ein Dialkylcarbonat eingesetzt wird, kann der abgespaltene Alkohol auch gemeinsam mit etwas Dialkylcarbonat aus dem Reaktor abdestilliert werden; außerhalb des Reaktors werden sodann Alkohol und Dialkylcarbonat getrennt, und das Dialkylcarbonat wird in die Reaktion zurückgeführt. Diese Trennung kann destillativ oder durch ein anderes Trennverfahren, beispielsweise durch Adsoption an einem Molekularsieb, erfolgen.

Die Abtrennung der heterogenen Titandioxidkatalysatoren nach erfolgter Umesterungsreaktion, kann beispielsweise durch Filtration, oder durch Zentrifugieren erfolgen, wobei auch inerte Verdünnungsmittel mitverwendet werden können, beispielsweise bei der Umsetzung zu oligomeren oder polymeren Produkten, Ebenso ist es möglich, die gebildeten Produkte direkt von den nicht flüchtigen Katalysatoren abzudestillieren, Die heterogenen Titankatalysatoren können auch in einem kontinuierlichen Umesterungsprozess in einem stationären Festbett als Kugeln, Ringe etc. eingesetzt werden, wobei natürlich eine besondere Katalysatorabtrennung nicht notwendig ist.

Beispiele

Eingesetzte Katalysatoren:

Katalysator I: PK 5585 (BAYER AG; feinteiliges Titandioxid-Pulver in Anatas-Modifikation mit einer BET-Oberfläche von 270 bis 330 $m^2/g$, und einem spezifischen Gewicht von 3,2 ± 0,1 $g/cm^3$) (erfindungsgemäß).

Katalysator II: PK 5585-1 (BAYER AG; feinteiliges Titandioxid-Pulver in Anatas-Modifikation mit einer BET-Oberfläche von 90 bis 110 $m^2/g$, und einem spezifischen Gewicht von 3,8 ± 0,1 $g/cm^3$) (erfindungsgemäß).

Katalysator III: Titandioxid-Pigment in Anatas-Modifikation mit einer BET-Oberfläche von 7 $m^2/g$ (zum Vergleich).

Katalysator IV: Titandioxid-Pigment in Rutil-Modifikation mit einer BET-Oberfläche von 7 $m^2/g$ (zum Vergleich).

Beispiele 1 bis 8

Allgemeine Versuchsvorschrift:

Zur Bestimmung der katalytischen Wirksamkeit des zu untersuchenden Katalysators unter vergleichbaren Bedingungen wurde eine Heißextraktionsapparatur, bestehend aus Mehrhalssumpfkolben, einem Extraktionsaufsatz (Rohr mit eingesetzter Extraktionshülse) und Rückflußkühler benutzt. Im Sumpfkolben wurde jeweils ein Gemisch aus 0,25 Mol Dialkylcarbonat und 0,50 Mol Phenol zum Sieden gebracht, so daß die leicht flüchtige Dialkylcarbonat-Komponente in den Extraktionsaufsatz destillierte und die mit jeweils 10 g Molsieb Zeolith A, 4 Å Porenweite (Baylith TE 144 der Fa. Bayer AG), gefüllte Extraktionshülse gleichmäßig durchströmte. Dabei stellte sich eine Reaktionstemperatur von 160°C ein. Durch Zugabe des zu untersuchenden Katalysators (je 5 g) zum Sumpfkolben, wurde die Reaktion in Gang gebracht (t = O), dabei der gebildete Reaktionsalkohol aus dem Reaktionsgemisch mit Hilfe des Dialkylcarbonats herausgeschleppt und am Molekularsieb dauerhaft gebunden. Durch Bestimmung der Produktbildung in Abhängigkeit von der Reaktionszeit durch gaschromatographische (GC) Analyse wurden die jeweiligen Reaktionsgeschwindigkeiten ermittelt.

Nach Beendigung des jeweiligen Umesterungsversuches wurden die Katalysatoren von den flüssigen Reaktionsprodukten durch Zentrifugieren abgetrennt, mehrfach mit Dichlormethan gewaschen, i. Vak. getrocknet und für einen weiteren Versuch eingesetzt.

Aus den Ergebnissen der Versuche 1 bis 8 sieht man deutlich die erheblich größere Aktivität der erfindungsgemäßen Titandioxid-Katalysatoren gegenüber den Standard-Anatas- bzw. -Rutil-Katalysatoren III u. IV. Nebenprodukte wurden im Gegensatz zu JP 54/125 617 nicht gefunden Insbesondere zeigte sich, daß die erfindungsgemäßen Katalysatoren ihre Aktivität auch nach längerem Gebrauch und wiederholtem Einsatz auf hohem Niveau beibehalten, während die Standard-Anatas- und -Rutil-Typen III und IV schon nach kurzer Zeit, d.h nach ein- bis zweimaliger Wiederverwendung, vollkommen desaktiviert sind.

Tabelle 1

| Beispiel | Katalysator | wieder verwendet | verwend.a) Dialkylcarbonat | t (h) | Produktbildung b) Gew.-% in Flächen % (GC) | |
|---|---|---|---|---|---|---|
| 1 erf.gem. | I | frisch | DEC | 4 | 9,0 EPC | 1,2 DC |
| | | 1 x | " | 4 | 7,0 " | 0,8 " |
| | | 2 x | " | 4 | 6,5 " | 0,8 " |
| | | 4 x | " | 4 | 6,5 " | 0,7 " |
| 2 erf.gem. | II | frisch | DEC | 4 | 4,0 EPC | 0,5 DC |
| | | 1 x | " | 4 | 3,0 " | 0,4 " |
| | | 2 x | " | 4 | 2,8 " | 0,4 " |
| | | 4 x | " | 4 | 2,8 " | 0,4 " |
| 3 z.Vgl. | III | frisch | DEC | 4 | 1,8 EPC | 0,05 DC |
| | | 2 x | " | 4 | 0,2 " | - |
| 4 z.Vgl. | IV | frisch | DEC | 4 | 0,6 EPC | 0,04 DC |
| | | 2 x | " | 4 | 0,1 " | - |
| 5 erf.gem. | I | frisch | DMC | 1 | 2,0 MPC | - |
| | | " | " | 4 | 11,8 " | 5,2 DC |
| 6 erf.gem. | II | frisch | DMC | 2 | 1,0 MPC | - |
| | | " | " | 4 | 3,0 " | - |
| 7 z.Vgl. | III | frisch | DMC | 2 | 0,4 MPC | - |
| | | " | " | 4 | 1,3 " | - |
| 8 z.Vgl. | IV | frisch | DMC | 2 | 0,1 MPC | - |
| | | " | " | 4 | 0,3 " | - |

a) DEC = Diethylcarbonat, DMC = Dimethylcarbonat,
b) DC = Diphenylcarbonat, EPC = Ethylphenylcarbonat, MPC = Methylphenylcarbonat,

## Patentansprüche

1. Verfahren zur Herstellung von mindestens eine aromatische Estergruppe enthaltenden Kohlensäurediestern aus mindestens eine aliphatische Estergruppe enthaltenden Kohlensäurediestern durch katalysierte Umesterung mit einem Phenol, dadurch gekennzeichnet, daß als Katalysator ein Titandioxid mit einer nach der BET-Methode bestimmten Oberfläche von mindestens 20 $m^2/g$ in einer Menge von 0,1 bis 200 Gew.-%, bezogen auf den eingesetzten, mindestens eine aliphatische Estergruppe enthaltenden Kohlensäurediester, eingesetzt wird.

2. Verfahren nach Anspruch 1, dadurch gekennzeichnet, daß als Katalysator ein Titandioxid mit einer Oberfläche mindestens 50 $m^2/g$, bevorzugt mindestens 90 $m^2/g$ eingesetzt wird.

3. Verfahren nach den Ansprüchen 1 und 2, dadurch gekennzeichnet, daß der Katalysator in Mengen von 1 bis 100 Gew.-%, bevorzugt 2 bis 50 Gew.-%, bezogen auf den eingesetzten, mindestens eine aliphatische Estergruppe enthaltenden Kohlensäurediester verwendet wird.

4. Verfahren nach Anspruch 1 bis 3, dadurch gekennzeichnet, daß mindestens eine aliphatische Estergruppe enthaltende Kohlensäurediester der Formel

$$R^1O\text{-}CO\text{-}OR^2$$

eingesetzt werden, in der

$R^1$ und $R^2$ unabhängig voneinander geradkettiges oder verzweigtes $C_1$-$C_{12}$-Alkyl oder $C_3$-$C_8$-Cycloalkyl bedeuten, wobei weiterhin $R^1$ substituiertes oder nicht substituiertes $C_6$-$C_{12}$-Aryl bedeuten kann.

5. Verfahren nach Anspruch 4, dadurch gekennzeichnet, daß mindestens eine aliphatische Estergruppe enthaltende Kohlensäurediester der Formel

$$R^3O\text{-}CO\text{-}OR^4$$

eingesetzt werden, in der

$R^3$ und $R^4$ unabhängig voneinander geradkettiges oder verzweigtes $C_1$-$C_8$-Alkyl, Cyclopropyl, Cyclopentyl oder Cyclohexyl bedeuten, wobei weiterhin $R^3$ substituiertes oder nicht substituiertes Phenyl bedeuten kann.

6. Verfahren nach Anspruch 1, dadurch gekennzeichnet, daß zur Umesterung ein Phenol der Formel

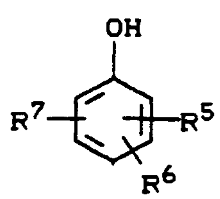

eingesetzt wird, in der

$R^5$ Wasserstoff, $C_1$-$C_4$-Alkyl, $C_2$-$C_4$-Alkenyl, $C_1$-$C_4$-Alkoxy, $C_5$-$C_6$-Cycloalkyl, Phenyl, Fluor, Chlor, Brom oder Cyano bedeutet;

$R^6$ für Wasserstoff, $C_1$-$C_4$-Alkyl, $C_2$-$C_4$-Alkenyl, $C_1$-$C_4$-Alkoxy, Fluor, Chlor, Brom steht und

$R^7$ Wasserstoff, $C_1$-$C_4$-Alkyl oder die Gruppe

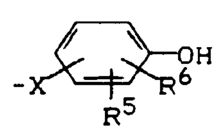

darstellt, in der X eine Einfachbindung, $-CH_2-$, $C_2$-$C_5$-Alkylen, $C_2$-$C_5$-Alkyliden, $C_5$-$C_6$-Cycloalkylen, $C_5$-$C_6$-Cycloalkyliden, Sauerstoff, Schwefel, -CO-, -SO- oder $-SO_2$-bedeutet, wobei $R^6$ und $R^7$ gemeinsam auch einen anellierten Benzolkern bedeuten können.

7. Verfahren nach Anspruch 7, dadurch gekennzeichnet, daß ein Phenol der Formel

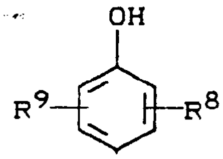

eingesetzt wird, in der

| | |
|---|---|
| $R^8$ und $R^9$ | unabhängig voneinander Wasserstoff, $C_1$-$C_4$-Alkyl oder Chlor bedeuten. |

**8.** Verfahren nach Anspruch 7, dadurch gekennzeichnet, daß ein Bisphenol der Formel

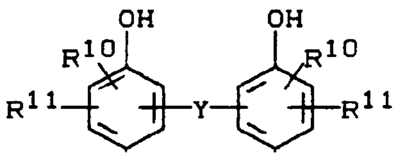

eingesetzt wird,
in der

| | |
|---|---|
| $R^{10}$ und $R^{11}$ | unabhängig voneinander Wasserstoff, $C_1$-$C_4$-Alkyl, $C_2$-$C_4$-Alkenyl, $C_1$-$C_4$-Alkoxy, $C_5$-$C_6$-Cycloalkyl, Fluor, Chlor oder Brom bedeuten kann, und |
| Y | für eine Einfachbindung, $-CH_2-$, $-C_2$-$C_5$-Alkyliden, $C_5$-$C_6$-Cycloalkyliden, Schwefel oder $-SO_2$-steht. |

**9.** Verfahren nach Anspruch 1, dadurch gekennzeichnet, daß bei einer Temperatur von 50-300°C, bevorzugt 100-250°C gearbeitet wird.

## Claims

**1.** Process for preparing diesters of carbonic acid containing at least one aromatic ester group from diesters of carbonic acid containing at least one aliphatic ester group by catalytic transesterification with a phenol, characterized in that the catalyst used is a titanium dioxide having a BET surface area of at least 20 $m^2/g$ in an amount from 0.1 to 200% by weight, based on the diesters of carbonic acid containing at least one aliphatic ester group which are used.

**2.** Process according to Claim 1, characterized in that the catalyst used is a titanium dioxide having a surface area of at least 50 $m^2/g$, preferably at least 90 $m^2/g$.

**3.** Process according to Claims 1 and 2, characterized in that the catalyst is used in amounts from 1 to 100% by weight, preferably from 2 to 50% by weight, based on the diesters of carbonic acid containing at least one aliphatic ester group which are used.

**4.** Process according to Claims 1 to 3, characterized in that diesters of carbonic acid containing at least one aliphatic ester group are used which have the formula

$$R^1O\text{-}CO\text{-}OR^2,$$

in which

| | |
|---|---|
| $R^1$ and $R^2$ | are, independently of one another, straight-chain or branched $C_1$-$C_{12}$-alkyl or $C_3$-$C_8$-cycloalkyl, where $R^1$ may furthermore be substituted or unsubstituted $C_6$-$C_{12}$-aryl. |

**5.** Process according to Claim 4, characterized in that diesters of carbonic acid containing at least one aliphatic ester group are used which have the formula

$$R^3O\text{-}CO\text{-}OR^4,$$

in which

| | |
|---|---|
| $R^3$ and $R^4$ | are, independently of one another, straight-chain or branched $C_1$-$C_8$-alkyl, cyclopropyl, cyclopentyl or cyclohexyl, where $R^3$ may furthermore be substituted or unsubstituted phenyl. |

6. Process according to Claim 1, characterized in that the transesterification is carried out using a phenol of the formula

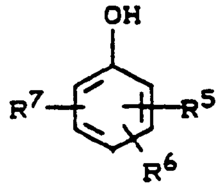

in which

$R^5$ is hydrogen, $C_1$-$C_4$-alkyl, $C_2$-$C_4$-alkenyl, $C_1$-$C_4$-alkoxy, $C_5$-$C_6$-cycloalkyl, phenyl, fluorine, chlorine, bromine or cyano;

$R^6$ represents hydrogen, $C_1$-$C_4$-alkyl, $C_2$-$C_4$-alkenyl, $C_1$-$C_4$-alkoxy, fluorine, chlorine, bromine and

$R^7$ represents hydrogen, $C_1$-$C_4$-alkyl or the group

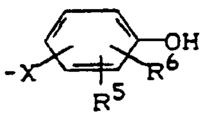

in which X is a single bond, $-CH_2-$, $C_2$-$C_5$-alkylene, $C_2$-$C_5$-alkylidene, $C_5$-$C_6$-cycloalkylene, $C_5$-$C_6$-cycloalkylidene, oxygen, sulphur, -CO-, -SO- or $-SO_2-$,
where $R^6$ and $R^7$ may together also be an anellated benzene nucleus.

7. Process according to Claim 7, characterized in that a phenol is used which has the formula

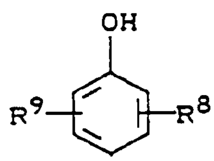

in which

$R^8$ and $R^9$ are, independently of one another, hydrogen, $C_1$-$C_4$-alkyl or chlorine.

8. Process according to Claim 7, characterized in that a bisphenol is used which has the formula

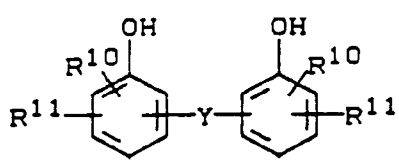

in which

$R^{10}$ and $R^{11}$ can be, independently of one another, hydrogen, $C_1$-$C_4$-alkyl, $C_2$-$C_4$-alkenyl, $C_1$-$C_4$-alkoxy, $C_5$-$C_6$-cycloalkyl, fluorine, chlorine or bromine, and

Y represents a single bond, $-CH_2-$, $-C_2$-$C_5$-alkylidene, $C_5$-$C_6$-cycloalkylidene, sulphur or $-SO_2-$.

9. Process according to Claim 1, characterized in that it is carried out at a temperature of 50-300°C, preferably 100-250°C.

## Revendications

1. Procédé de préparation de diesters carboniques contenant au moins un groupe ester aromatique à partir de diesters carboniques contenant au moins un groupe ester aliphatique par transestérification avec un phénol en présence d'un catalyseur, caractérisé en ce que l'on utilise en tant que catalyseur un dioxyde de titane ayant une surface, déterminée par la méthode BET, d'au moins 20 $m^2/g$ en quanttié de 0,1 à 200% du poids du diester carbonique mis en oeuvre, contenant au moins un groupe ester aliphatique.

2. Procédé selon la revendication 1, caractérisé en ce que l'on utilise en tant que catalyseur un dioxyde de titane ayant une surface d'au moins 50 $m^2/g$ et de préférence d'au moins 90 $m^2/g$.

3. Procédé selon les revendications 1 et 2, caractérisé en ce que l'on met en oeuvre le catalyseur en quantité de 1 à 100% en poids, de préférence de 2 à 50% du poids du diester carbonique mis en oeuvre, contenant au moins un groupe ester aliphatique.

4. Procédé selon les revendications 1 à 3, caractérisé en ce que l'on met en oeuvre des diesters carboniques contenant au moins un groupe ester aliphatique et répondant à la formule :

$$R^1O\text{-}CO\text{-}OR^2$$

dans laquelle

$R^1$ et $R^2$ représentent chacun, indépendamment l'un de l'autre, un groupe alkyle en $C_1$-$C_{12}$ à chaîne droite ou ramifiée ou un groupe cycloalkyle en $C_3$-$C_8$, $R^1$ pouvant en outre représenter un groupe aryle en $C_6$-$C_{12}$ substitué ou non.

5. Procédé selon la revendication 4, caractérisé en ce que l'on met en oeuvre des diesters carboniques contenant au moins un groupe ester aliphatique et répondant à la formule :

$$R^3O\text{-}CO\text{-}OR^4$$

dans laquelle

$R^3$ et $R^4$ représentent chacun, indépendamment l'un de l'autre, un groupe alkyle à chaîne droite ou ramifiée en $C_1$-$C_8$, un groupe cyclopropyle, cyclopentyle ou cyclohexyle, $R^3$ pouvant également représenter un groupe phényle substitué ou non.

6. Procédé selon la revendication 1, caractérisé en ce que l'on met en oeuvre pour la transestérification un phénol de formule :

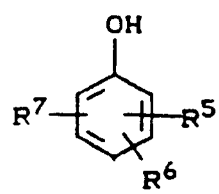

dans laquelle

$R^5$ représente l'hydrogène, un groupe alkyle en $C_1$-$C_4$, alcényle en $C_2$-$C_4$, alcoxy en $C_1$-$C_4$, cycloalkyle en $C_5$-$C_6$, phényle, le fluor, le chlore, le brome ou un groupe cyano ;

$R^6$ représente l'hydrogène, un groupe alkyle en $C_1$-$C_4$, alcényle en $C_2$-$C_4$, alcoxy en $C_1$-$C_4$, le fluor, le chlore, le brome et

$R^7$ représente l'hydrogène, un groupe alkyle en $C_1$-$C_4$ ou le groupe

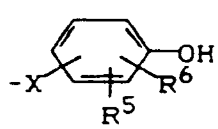

pour lequel

X représente une liaison simple, un groupe $-CH_2-$, alkylène en $C_2$-$C_5$, alkylidène en $C_2$-$C_5$, cycloalkylène en $C_5$-$C_6$, cycloalkylidène en $C_5$-$C_6$, l'oxygène, le soufre, un groupe -COO-,

| | |
|---|---|
| | -SO- ou $-SO_2-$, |
| $R^6$ et $R^7$ | pouvant également représenter ensemble un noyau benzénique condensé. |

7. Procédé selon la revendication 7, caractérisé en ce que l'on met en oeuvre un phénol de formule :

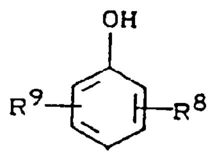

dans laquelle

| | |
|---|---|
| $R^8$ et $R^9$ | représentent chacun, indépendamment l'un de l'autre, l'hydrogène, un groupe alkyle en $C_1$-$C_4$ ou le chlore. |

8. Procédé selon la revendication 7, caractérisé en ce que l'on met en oeuvre un bisphénol de formule :

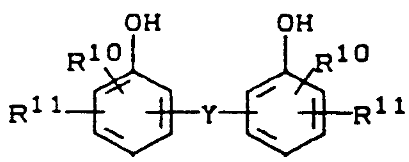

dans laquelle

| | |
|---|---|
| $R^{10}$ et $R^{11}$ | représentent chacun, indépendamment l'un de l'autre, l'hydrogène, un groupe alkyle en $C_1$-$C_4$, alcényle en $C_2$-$C_4$, alcoxy en $C_1$-$C_4$, cycloalkyle en $C_5$-$C_6$, le fluor, le chlore ou le brome et |
| Y | représente une liaison simple, un groupe $-CH_2-$, alkylidène en $C_2$-$C_5$, cycloalkylidène en $C_5$-$C_6$, le soufre ou le groupe $-SO_2-$. |

9. Procédé selon la revendication 1, caractérisé en ce que l'on opère à une température de 50 à 300°C, de préférence de 100 à 250°C.